Europäisches Patentamt

European Patent Office

Office européen des brevets

(11) Publication number: **0 078 114**

**A1**

# EUROPEAN PATENT APPLICATION

(21) Application number: **82305278.2**

(22) Date of filing: **04.10.82**

(51) Int. Cl.³: **A 61 L 9/12**
**B 65 D 25/38**

(30) Priority: **26.10.81 US 315535**

(43) Date of publication of application:
**04.05.83 Bulletin 83/18**

(84) Designated Contracting States:
**AT BE CH DE FR GB IT LI LU NL SE**

(71) Applicant: **KAUFMAN, John George**
**858 Condor Drive Burlington**
**Ontario L7T 3A7(CA)**

(72) Inventor: **KAUFMAN, John George**
**858 Condor Drive Burlington**
**Ontario L7T 3A7(CA)**

(74) Representative: **Connor, Terence Kevin et al,**
**FITZPATRICKS Kern House 61/62 Lincoln's Inn Fields**
**London WC2B 6EX(GB)**

(54) **Liquid feed device.**

(57) A liquid feed device, e.g. a room deodorizer, a plant feeder or a dispenser for a toilet bowl cleanser, has a main body (26) of liquid in an upwardly closed container (11) and an air chamber structure (20) within the container and in communication with the body (26) of liquid to allow the liquid to flow into the air chamber structure (20) to form therein an auxiliary body (27) of liquid beneath an air space (31) in the structure (20). An outlet passage (16) extends from the air space (31) to the exterior of the chamber (20) and a wick (w) or other device is provided within the air chamber structure (12) for continuously transferring liquid from the auxiliary body (27) of liquid, through the outlet passage (16) to the exterior of the container (11). A partial vacuum in the container (11) above the main body (26) of liquid limits the level of the auxiliary body (27) of liquid.

FIG. 1

Croydon Printing Company Ltd

The present invention relates to a liquid feed device and is useful in particular, but not exclusively, for room deodorizers, plant feeders, intravenous drip feeds, etc.

One type of room deodorizer which is commercially available at the present time comprises a wick which can be pulled upwardly through the top of a container holding a supply of deodorizer liquid, so that an upper portion of the wick becomes exposed to the atmosphere, while a lower portion of the wick remains immersed in the liquid within the container. The liquid is drawn up the wick by a wicking action to the upper portion of the wick, where it is gradually evaporated into the atmosphere. As more and more of tne liquid within tne container is consumed in this way, and the level of the liquid within the container consequentially falls, the rate at which the liquid is drawn upwardly along the wick and evaporated from the upper portion of the wick is correspondingly reduced.

Other devices, such as drip feed devices, which rely upon a wick for drawing liquid from a body of liquid suffer from the same disadvantage that the rate at which the liquid is drawn off varies, i.e. reduces, as the level of the body of liquid falls.

United States Patent 1,596,791, issued August 17, 1926 to A.L. Ball and United States Patent 1,349,703 issued August 17, 1920, disclose devices in which a container is positioned upside down with a neck of the container in a cup-like liquid receiver from which a wick extends, so that liquid from the container flows into the receiver until the liquid level reaches the mouth of the container neck and so that the wick dispenses liquid from the receiver by a wicking action. However, these prior devices have the disadvantage that there is a risk of spillage of the contents of the containers as they are inverted to position their necks in the receivers.

In the United States Patent 3,157,319, issued November 17, 1964 to G. Schwienbacher, there is disclosed a dispenser for easily dripping liquids which comprises a container having an overflow duct extending through the bottom of the container from the interior of a hood in the container, the interior of the container communicating through ducts with the interior of the hood and the container being resiliently deformable for the expulsion of liquid from the container through the hood and through the overflow duct. However, the dispenser disclosed in the aforesaid United States Patent 3,157,319 is intended to expel liquid only on deformation of the container and not in a continuous manner.

It is according an object of the present invention to provide a novel and improved liquid feed device which is simple to handle and provides an at least approximately constant liquid level from which liquid can be drawn off by a wick or other liquid adsorber.

According to the present invention, there is provided a liquid feed device, comprising an upwardly closed container; a main body of liquid in the container; means defining an air chamber; means providing communication between a bottom region of the air chamber and the body of liquid to allow the liquid to flow into the air pocket chamber defining means and to form an auxiliary body of the liquid beneath an air space in the chamber; means defining an outlet passage extending from the air space to the exterior of the container; and means for continuously transferring liquid from the auxiliary body of liquid and through the outlet passage to the exterior of the container.

In operation of the present invention, the air chamber provides an at least approximately constant level of the liquid within the air chamber, and thus reduces variations in the rate of discharge of the liquid through the outlet passage. This liquid level may vary somewhat in response to changes in atmospheric temperature and pressure and such variations may be counteracted, for example, by supporting a wick on a float within the air chamber.

Embodiments of the invention are described below with reference to the accompanying drawings, in which:-

Figures 1 and 2 show views taken in vertical, cross-section through first and second drip feed devices embodying the present invention;

Figure 3 shows a corresponding view through a room deodorizer device according to a third embodiment of the present invention;

Figure 4 shows a corresponding view through a room deodorizer device provided with a floatingly supported wick according to a further embodiment of the present invention;

Figure 5 shows a corresponding view through a room deodorizer device provided with an induced air flow according to a further embodiment of the present invention;

Figure 6 shows a corresponding view through a drip feed device according to a further embodiment of the present invention, in which the wick is replaced by a liquid adsorbant material;

Figure 7 shows a corresponding view through a room deodorizer device according to a further embodiment of the present invention, having an upstanding wick end portion from which liquid is evaporated;

Figure 8 shows a corresponding view through a further embodiment of the present invention;

Figure 9 shows a view in vertical cross-section through a float; and

Figures 10 and 11 show a view in vertical cross-section through two further embodiments of the invention.

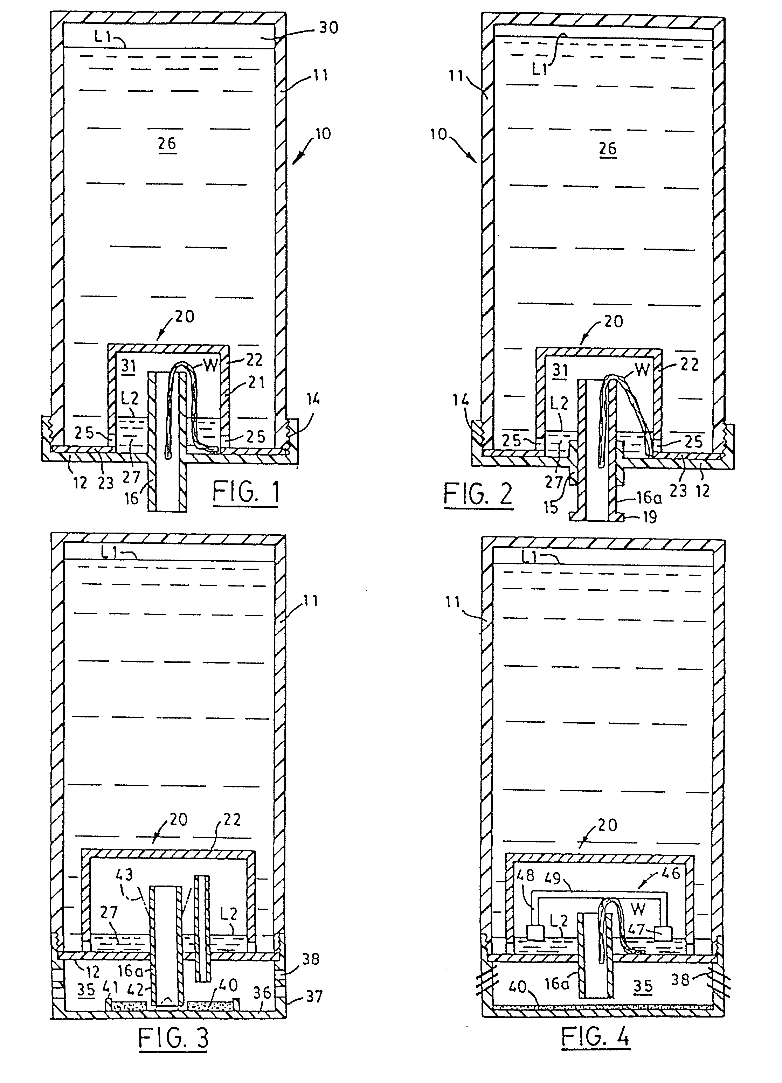

The drip feed device illustrated in Figure 1 of the accompanying drawings and indicated generally by reference numeral 10 comprises an open-bottomed container 11 provided with a bottom closure 12, which is secured to the container 11 by screw threads 14. The container 11 and the bottom closure 12 are of molded plastics material and the screw threads 14 or other suitable means provide a liquid-tight seal between the interengaged portions of the container 11 and the bottom closure 12.

The bottom closure 12 is formed with an integral vertical outlet tube indicated generally by reference numeral 16.

Within the container 11, there is provided an insert in the form of an air chamber structure indicated generally by reference numeral 20. The air chamber structure 20 comprises a cylindrical wall 21, closed at the top thereof by a flat top wall 22, and an annular flange 23 projecting laterally outwardly from the lower end of the cylindrical wall 21. The outer periphery of the annular flange 23 is clamped between the bottom closure 12 and the lower edge of the container 11.

Adjacent the annular flange, the cylindrical wall 21 of the air chamber structure 20 is formed with a plurality of openings 25, which provide communication between the interior of the air chamber structure 20 and a supply of liquid 26 provided within the container 11 at the exterior of the air chamber structure 20. The level of the liquid 26 within the container 11 is indicated by reference L1 and the liquid 26 flows through the openings 25 into the air chamber structure 20 to provide, within the air chamber structure 20, a secondary or auxiliary body of the liquid, which is indicated by reference numeral 27 and the level of which is indicated by reference L2.

Since the threads 14 provide an airtight connection between the bottom closure 12 and the container 11, a partial vacuum exists within the container 11 above the liquid level L1 and, therefore, the main body of liquid 26 is prevented from flowing out of the container 11 through the outlet tube 16. More particularly, if the pressure in the space 30 above the liquid level L1 within the container 11 is represented by P, then the following holds true:-

$$H \times sg + P = \text{atmospheric pressure}$$

Where H represents the difference between the liquid levels L1 and L2 and sg represents the specific gravity of the liquid. Since the interior of the air pocket structure 20 communicates with the atmosphere through the outlet passage 16, atmospheric pressure will prevail in an air space 31 defined by the air chamber structure 20 above the liquid level L2 and the level L2 of the secondary body of liquid 27 within the air chamber structure 20 will not be able to rise unless the pressure P is increased.

A wick W extends from the secondary body of liquid 27 through the air space 31 into the top of the outlet tube 16 and downwardly along the interior of the outlet tube 16 and serves to draw liquid from the secondary body of liquid 27 and to discharge it in the form of drips through the outlet tube 16.

Alternatively, the wick W may be replaced by a capillary tube arrangement or a coating of material, e.g. blocking, on the outlet tube or the inner surface of the air chamber structure 20, the material being such as to cause liquid from the auxiliary body of liquid 27 to rise and wet the material and thus to trickle down the outlet tube.

When a wick or capillary tube structure is employed, it may extend through, or even be incorporated in, the wall of the outlet tube.

The pressure P in the space 30 may vary in response to temperature and pressure changes in the environment, and consequentially the liquid level L2 may fluctuate somewhat.

These liquid level fluctuations will cause corresponding variations in the immersion of the wick W in the secondary body of liquid 27, and will consequentially vary somewhat the rate of discharge of the liquid through the outlet passage 16.

Such variations can be counteracted by the modification illustrated in Figure 2, in which the outlet tube 16 has been replaced by an outlet tube 16a which is slidably engaged, in a liquid-tight manner, in a sleeve 15 which is integral with the bottom closure 12. By sliding the outlet tube 16a into or out from the air chamber structure 20 to a greater or lesser extent, the position of the wick W can be varied and thereby the rate of discharge can be adjusted, and an annular rim 19 is provided at the lowermost end of the outlet tube 16a to facilitate manual engagement of the outlet tube 16a for this purpose. In the same way, variations in atmospheric pressure can be compensated. Apart from these atmospheric temperature and pressure variations, the liquid level L2 will remain substantially constant and, therefore, the rate of discharge of the liquid through the outlet tube 16a will likewise remain substantially constant but can be varied, as desired, by sliding adjustment of the outlet tube 16a.

The rate of discharge of the liquid could alternatively be varied by making the wick W adjustable independently of the outlet tube 16 or 16a.

Various other modifications and applications of the drip feed device described above with reference to Figures 1 and 2 are illustrated in the remaining Figures, in which the references shown in Figures 1 and 2 have been employed to indicate similar parts.

In the room deodorizer of Figure 3, the outlet tube 16a is formed in one piece with the bottom closure 12, as in Figure 2, and is therefore not adjustable inwardly and outwardly of the air chamber structure 20.

In this embodiment of the invention, an airflow chamber 35, defined by a bottom wall 36 and a cylindrical side wall 37 provided with openings 38, is provided below the bottom closure 12 for supporting an annular pad 40 of liquid adsorbant material. The annular pad 40 is provided within a cylindrical wall 41 upstanding from the bottom wall 36 and defining therewith an open-topped receptacle for receiving the liquid dripping downwardly from the outlet tube 16a.

The lower end of the outlet tube 16a is below the top of the wall 41 and, indeed, below the top of the annular pad 40. Consequentially, when an excessive amount of the liquid is discharged from the outlet tube 16 and had saturated the pad 40, it will be retained by the annular wall 41 and will accumulate in the central opening of the annular pad 40 until it reaches the bottom of the outlet tube 16 and thus forms a closure across the bottom of the outlet tube 16 for preventing the discharge of further liquid from the outlet tube 16. Such discharge will then not reoccur until sufficient of the liquid in the air flow chamber 35 has evaporated to free the bottom of the outlet tube 16. The liquid which evaporates in the air flow chamber 35 is free to defuse into the surrounding atmosphere through the louvered openings 38. A second outlet tube 42 is provided, without a wick, for relieving the air pressure within the air chamber structure 20 when the ambient temperature rises. However, the outlet tube 42 may be omitted if not required.

If desired, the bottom wall 36 may be provided, in alignment with the outlet tube 16, with a raised projection or dimple 42 to facilitate such blockage of the outlet tube 16 by the liquid accumulated at the lower end of the outlet tube 16.

It may occasionally be desired to discharge a larger than usual amount of the liquid from the container 11 into the air flow chamber 35. This can be achieved by inverting the deodorizer and then restoring it to its normal position, so that the liquid in the air chamber structure 20 is firstly caused to flow towards the wall 22 and then to flow down the outlet tube 16a. To facilitate this, the uppermost end of the outlet tube 16a may be formed with a funnel portion as indicated by reference numeral 43.

Alternatively, the container 11 may be made of a flexible material to allow the container 11 to be squeezed for expelling some of the liquid from the container 11, in which case the air chamber structure 20 may be made of rigid material and dimensioned so as to limit the amount by which the container 11 can be squeezed and thus to limit the amount of liquid expelled.

In the embodiment of Figure 3, the annular pad 40 of Figure 2 is replaced by a circular pad 45 of liquid adsorbant material for adsorbing and evaporating the liquid discharged from the outlet tube 16.

In addition, in this embodiment the wick W is supported by a float device, indicated generally by reference numeral 46, which floats in the secondary body of liquid 27 within the air chamber structure 20 and thus maintains the wick W at a constant level relative to the level of the liquid, regardless of fluctuations in the liquid level L2. The flow device 46 comprises a plurality of floats 47 provided at the lower ends of arms 48 of a spider 49, to which the wick W is attached.

Other parts of the deodorizer shown in Figure 4 which correspond to those of Figure 3 have been indicated by the same reference numerals.

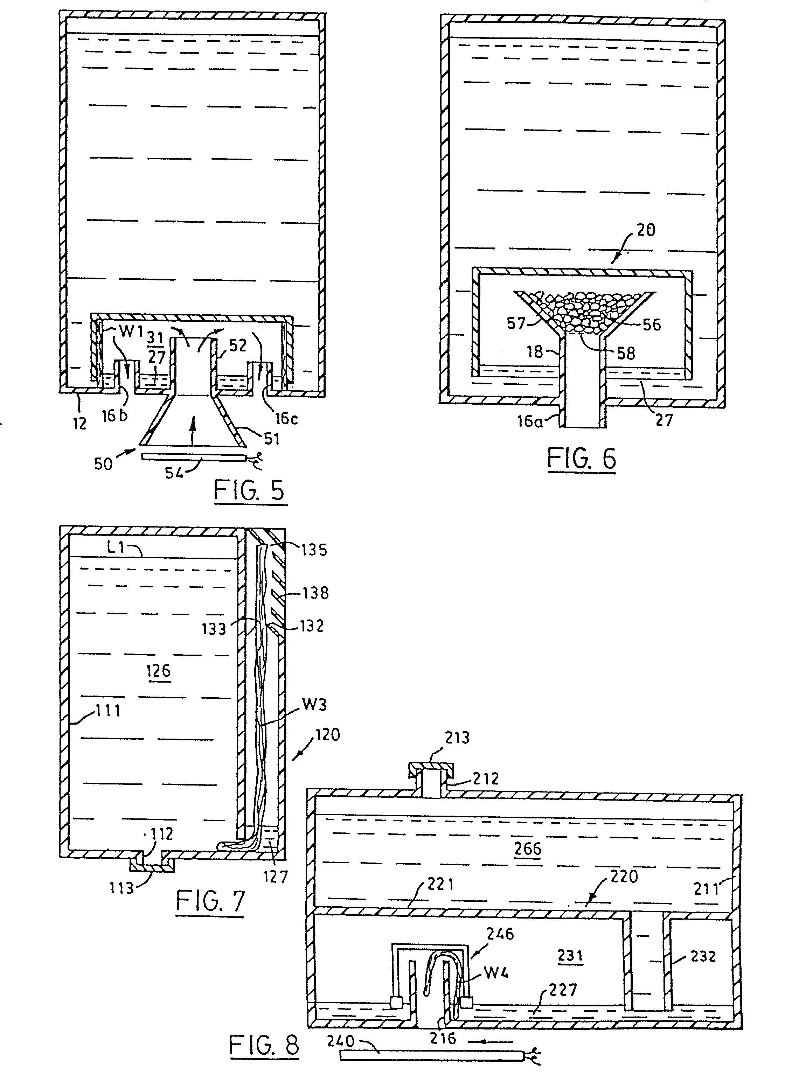

In the embodiment of the invention illustrated in Figure 5, the single outlet tube 16 of Figure 1 is replaced by a pair of outlet tubes 16b and 16c, which are offset from the centre of the bottom closure 12. The outlet tubes 16b and 16c could alternatively be replaced by one or more outlet tubes extending from the air space 31 to the exterior of the container 11 through the side or top of the container 11.

A hot air inlet passage, indicated generally by reference numeral 50 and comprising a lower, funnel-shaped portion 51 and a cylindrical upper portion 52, extends through the centre of the bottom closure 12 into the air pocket chamber 31 defined by the air pocket structure 20.

The wick W of Figures 1 to 4 is replaced by a cylindrical wick W1 extending around the interior surface of the side wall of the air chamber structure and adjacent the outlet tubes 16b and 16c.

In addition, an electrical heater element 54 is positioned below the funnel-shaped bottom portion 51 of the air inlet passage 50.

In operation of this embodiment, the heater element 54 is electrically energized, from a suitable source of electrical power (not shown), so as to produce an upwardly flowing stream of convection air, as indicated by the arrows, which flows into the air pocket chamber 31, over the wick W1 and downwardly through the outlet tubes 16b and 16c. This air causes evaporation of the liquid from the wick W1, which extends downwardly into the secondary body of liquid 27, and entrains the evaporated liquid through the outlet tubes 16b and 16c since the wick is always fully saturated variations in the level of the auxiliary body of liquid 27 do not affect the amount of vapour discharge to the atmosphere.

The wick W may be replaced by a coating of material on the interior wall of the air chamber structure, the material being such as to cause the liquid to rise up and wet the interior wall.

Instead of employing a heater to produce an air flow as described above, it would of course alternatively be possible to employ a fan or the like. A further possibility is to rely on convection air currents resulting from temperature differences between the interior of the air chamber structure and the ambient temperature.

Figure 6 shows an embodiment of the invention in which the wick W of Figure 1 is replaced by a liquid-adsorbant material 56, which is supported in a funnel-shaped upper end portion 57 of the outlet tube 16 by a suitable perforated support or screen 58 extending across the upper end of the cylindrical outlet tube portion 18. In operation of this embodiment, liquid is adsorbed from the secondary body of liquid 27 by the liquid adsorbant material 56 and dripped downwardly therefrom through the screen 58 and the outlet tube 16.

In Figure 7, there is shown a room deodorizer comprising a container indicated generally by reference numeral 111 and provided, at its bottom, with a filling spout 112 provided with an airtight closure cap 113. The container holds a body of liquid 126 having an upper level L1, and has, at one side thereof, an air chamber structure indicated generally by reference numeral 120 defining an air space 131 and containing a wick W3. The top of the air space 131 is sealed to the wick by means of a rubber or plastic seal 132, and the wick W3 has an uppermost portion 133 projecting upwardly beyond the seal 132 into an air flow chamber 135, which is open to the atmosphere through louvers 138.

Liquid flows from the body of liquid 126 to form a secondary body of liquid 127 at the bottom of the air space 131. Comparison of Figure 1 and 7 will make it readily apparent that the embodiment of Figure 7 operates in a manner analogous to that of the embodiment of Figure 1, except that in the case of Figure 7 the liquid drawn along the wick W3 by a wicking action is not dripped downwardly through an outlet tube corresponding to the outlet tube 16, but rather is exposed, in the air flow chamber 135, to evaporation from the upper end portion 133 of the wick W3. However, the liquid level of the secondary body of liquid 127 is maintained at least approximately constant in the same manner as described above with reference to Figure 1.

Figure 8 shows a deodorizer comprising a housing tube 211 provided, at its top, with a filling spout 212 having an airtight closure cap 213.

An air chamber structure, indicated generally by reference numeral 220, is formed in the container 211 by means of a horizontal wall 221 defining an air space 231 within the housing 211, the wall 221 and the air space 231 being interrupted by a tube 232 forming a liquid duct extending downwardly from the wall 211 to a position adjacent the bottom of the container 211, which is indicated by reference numeral 233. As will be apparent from comparison of Figures 1 and 8, the air chamber structure 220 corresponding to the air chamber structure 20 of Figure 1, the liquid duct 232 providing communication between a body of liquid 226 in the container and the interior of this air chamber structure, with a secondary body of liquid 227 in the air chamber structure corresponding to the secondary body of liquid 27 of Figure 1.

A wick, indicated by reference W4 and corresponding to wick W of Figure 1, is supported on a float structure 246 corresponding to the float structure 46 of Figure 3, so that the wick W4 is kept at constant level relative to the level of the secondary body of liquid 227, irrespective of changes of the level of the latter. The wick W4 extends into an outlet tube 216, corresponding to the outlet tube 16 of Figure 1, and terminates above a pad of adsorbant material 240, so that liquid dripping from the wick W4 onto the pad 240 is exposed on the pad 240 to evaporation.

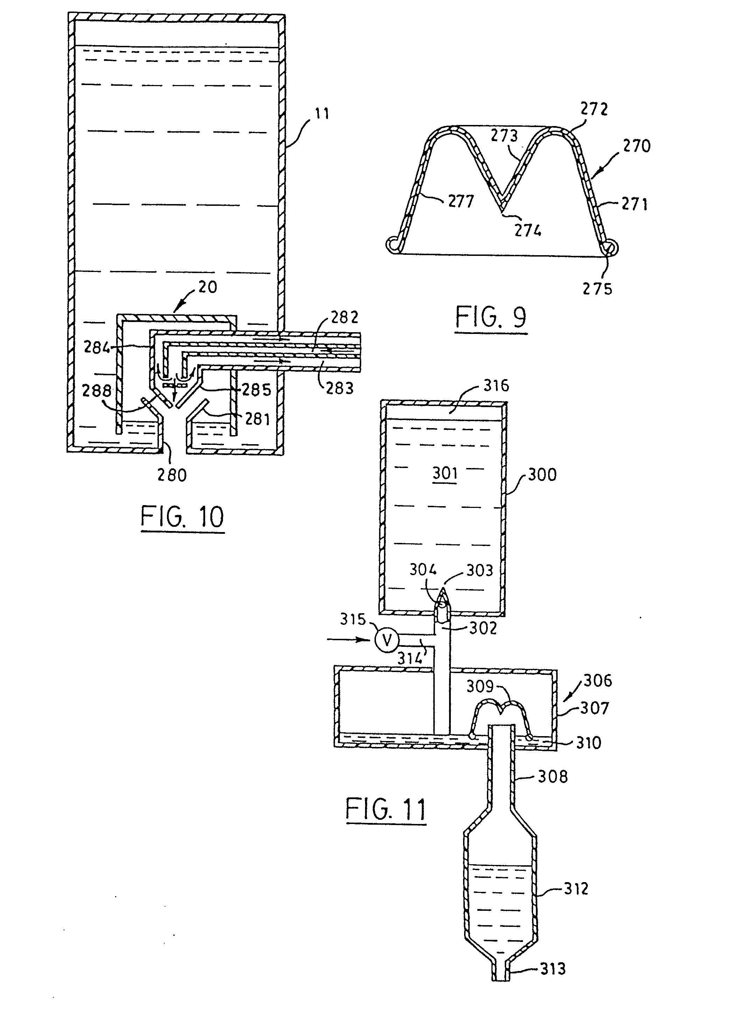

Figure 9 shows a float, indicated generally by reference numeral 270, which may be employed in place of the float device 46 and the wick W of the deodorizer shown in Figure 3.

The float 270 is the form of a downwardly-open shell having a frusto-conical wall 271 which merges smoothly through an annular top 272 of upwardly-convex shape with a central, downwardly projecting conical portion 273 terminating at a downwardly directed tip or point 274.

A tubular annular rim 275 extends around the lower periphery of the frusto-conical wall 271 and is formed in one piece therewith.

The interior of the float 270 is covered by a coating 277 of a material which causes the liquid in the container to rise along the inner surface of the float 270 so as to drip downwardly from the tip 274.

In use, the float 270 is inserted into the air chamber structure 20, with the tubular annular rim 275 floating on the surface of the auxiliary body of liquid within the air chamber structure 20 and with the tip 274 positioned in or above the top of the outlet tube 16. The float 270 thus rises or falls with

the level L2 of the auxiliary body of liquid, so that a constant rate of dripping from the tip 274 is ensured. The rim 274 serves as a stabilizer for stabilizing the floating of the float 270, which is therefore uniformly immersed in the liquid around the lower periphery of the float 270.

Instead of employing the float 270, it is alternatively possible, as mentioned previously, to provide a coating on the inner surface of the air pocket structure or the outer surface of the outlet tube 16 which will cause the liquid to rise and enter the top of the outlet tube 16 and, when the air pocket structure is employed for this purpose, a suitable formation may be provided in the top of the air pocket structure 20, e.g. similar to the tip 274 of Figure 9, to encourage dripping of the liquid into the top of the outlet tube 16.

Figure 10 illustrates an alternative embodiment of the invention in which condensation is utilized to produce dripping of the liquid from the container.

In this case, the container 11 is provided, at its bottom, with an outlet tube 280 formed, at the top thereof, with a funnel portion 281, the tube 280 and its funnel portion 281 being disposed within the air pocket structure 20.

Coaxial air flow passages 282 and 283 extend through the side wall of the container 11 into the air pocket structure 20 to respective inner and outer vertical tubes 284 and 285, which are located in the air pocket within the air chamber structure 20.

The inner tube 284 is formed with openings at its lower end, so that, as indicated by arrows, air flowing into the container 11 through the inner air flow passage 282 can exist from the inner tube 284 and flow from the outer tube 285 along the outer air flow passage 283 to the exterior of the container 11.

The outer tube 285 has a downwardly-pointed conical bottom 286 which is located within the funnel portion 281 of the outlet tube 280.

In operation of this embodiment, cold air is passed along the inner air flow passage 282 for flow as indicated by the arrows and as described above, and this cold air cools the outer tube 285 and the bottom 286 of the outer tube 285, so as to produce condensation, on the outer surface thereof, of vapour of the liquid within the air pocket structure 20.  This condensate then drips downwardly through the outlet tube 280.

Figure 11 shows an intravenous feed arrangement comprising a container 300 for a main body of liquid 301, the container 300 being in form of an air tight bag or poach of plastic material.

A tube 302 having a pointed tip 303 and an opening 304 in the wall of the tube 302 adjacent the tip 303 is inserted into the container 300, by piercing of the tip 303 through the plastic wall of the container 300, to allow the liquid to flow from the main body of liquid 301 into the interior of the tube 302.

The tube 302 extends downwardly through the top of an air chamber structure, indicated generally by reference numeral 306, which is formed by an upwardly-closed housing 307, the tube 302 extending downwardly through the top of the housing 307 to a position spaced from but close to the bottom of the housing 307.

An outlet tube 308 extends downwardly through the bottom of the housing 307, and a float 309, which is similar to the float 270 of Figure 9, is bouyantly supported on an auxiliary body 310 of the liquid within the housing 307.

The outlet tube 308 extends downwardly to a transparent bottle 312, from the bottom of which extends a tube 313 for intravenous connection to the patient.

An air inlet passage 314 communicates with the tube 302 and is provided with a valve 315 for controlling the entry of air through the air inlet tube 314, the tube 302 and the opening 304 into the interior of the container 300.

Above the body of liquid 301 in the container 300, there is a space 316 which is occupied by a partial vacuum and, as will be readily apparent, the liquid cannot flow from the container 300 through the outlet tube 302, when the level of the auxiliary body 310 of liquid is as high as the bottom of the outlet tube 302, until the valve 315 is actuated to allow air to enter the container 300.

In operation of this arrangement, the valve 315 is actuated to raise the level of the auxiliary body 310 of liquid, but without allowing it to reach the top of the outlet tube 308. The liquid is then dripped at a constant rate from the float 309 into the outlet tube 308.

As compared with prior art deodorizers and the like employing long, large wicks, the above-described embodiments of the invention which employ a wick have the advantage that the wick can be short and of small cross-sectional size.

Furthermore the present invention provides a more accurate and more economic means for continuously dispensing a liquid than has been provided hitherto and enables this to be achieved by a self-contained device which avoids, for example, the provision of a liquid container and a wick-containing device separate from the container.

CLAIMS

1.    A liquid feed device, comprising:-

a main body (26) of liquid in an upwardly closed container(11), a passage (25) providing communication between a bottom region of an air chamber (20) in the container (11) and the body of liquid to allow the liquid to flow into the air chamber (20) and to form an auxiliary body (27) of the liquid beneath an air space (31) in the chamber, and an outlet passage (16) extending from the air space (31) to the exterior of the container (11), characterized by means (W) for continuously transferring liquid from the auxiliary body (27) of liquid and through the outlet passage (16) to the exterior of the container (11).

2.    A liquid feed device as claimed in claim 1, characterized in that the liquid transferring means comprise wick means (W) extending from the auxiliary body of liquid (27) to the outlet passage (16) for transferring the liquid to the outlet passage (16) by a wicking action.

3.    A liquid feed device as claimed in claim 2, characterized in that the outlet passage (16) extends downwardly from the air space (131), and the wicking means (W) extends into the outlet passage (16).

4.    A liquid feed device as claimed in claim 3, characterized by means (15, 16a) for varying the amount of wick means (W)immersed in the auxiliary body (31) of liquid to thereby vary the rate of discharge of the liquid through the outlet passage (11).

5.        A liquid feed device claimed in claim 3, characterized by means (48, 246, 270, 309) for maintaining the wick means at a constant level relative to the surface of the auxiliary body (31) of liquid to maintain a substantially constant rate of discharge of the liquid through the outlet passage (16) during variations of the surface level of the auxiliary body (31) of liquid.

6.        A liquid feed device as claimed in claim 5, characterized in that the wick-level maintaining means comprise float means (48, 246, 270, 309) bouyantly supported on the auxiliary body (31) of liquid for carrying the wick means (W) W4, 277).

7.        A liquid feed device as claimed in claim 6, characterized in that the wick means comprises a coating (277) of material on the float means (270).

8.        A liquid feed device as claimed in claim 2, characterized by means (15) for adjustably supporting the outlet passage (16) relative to the container (11), the wick means (W) being supported for displacement with the outlet passage (16) to vary the immersion of the wick means (W) in the auxiliary body (27) of liquid and thereby to vary the rate of discharge of the liquid through the outlet passage (16).

9.        A liquid feed device as claimed in claim 1, characterized by means (40) for adsorbing the liquid discharged from the outlet passage (16), (36), means for supporting the liquid adsorbing means (40) below the outlet passage (16) and means (38) defining an air passage for communication of atmospheric air with the liquid adsorbing means (40).

10.    A liquid feed device as claimed in claim 1, characterized by means (40) for receiving the liquid discharged from the outlet passage (16a), the outlet passage (16a) having a lower end extending into the liquid receiving means (40), whereby the outlet passage (16a) becomes blocked at the lower end by the discharged liquid when a sufficient amount of the discharged liquid has collected in the receiving means (40).

11.    A liquid feed device as claimed in claim 10, characterized in that the outlet passage defining means comprise a tube (16a) extending downwardly from the air space (31) and the liquid receiving means comprise an upwardly open receptacle (36, 41) located below the tube (16a), the receptacle having an upstanding wall (41) extending around and spaced from the lower end.

12.    A liquid feed device as claimed in claim 11, characterized by a liquid adsorbant material (40) in the liquid receptacle (36, 41).

13.    A liquid feed device as claimed in claim 1, further including means for exerting a pressure on the liquid supply (26) in the container (11) to discharge a portion of the liquid supply through the outlet passage (16, 16a, 216).

14.    A liquid feed device as claimed in claim 1, characterized in that the container (11) has a variable volume to permit expulsion of a portion of the liquid supply (26) from the container (11) through the outlet passage (16) by pressure exerted on the container (11).

15.     A liquid feed device as claimed in claim 1, characterized in that the container (11) comprises a squeeze bottle having a wall which is flexibly squeezable for expelling a portion of the liquid supply (26) through the outlet passage (16).

16.     A liquid feed device as claimed in claim 1, characterized in that the air chamber (20) defining means are spaced from the wall and adapted to limit the squeezing of the container (11).

17.     A liquid feed device as claimed in claim 1, characterized in that the liquid transferring means comprise wick means (W) extending into the auxiliary body (27) of liquid for raising liquid therefrom by a wicking action, the wick means (W) being spaced from the outlet passage (16b, 16c) within the air space (31), and means (54) for conducting an air flow past the wick means (W) and through the outlet passage (16b, 16c).

18.     A liquid feed device as claimed in claim 17, characterized in that the air flow conducting means comprise means defining an air inlet passage (52) extending into the air space (31) from the exterior of the container.

19.     A liquid feed device as claimed in claim 18, characterized by means (54) for producing a flow of air into the air space (31) through the air inlet (52).

20.     A liquid feed device as claimed in claim 1, characterized in that the liquid transferring means comprise means (56) for absorbing liquid from the auxiliary body (27) of liquid and means (57) supporting the liquid adsorbant means (56) above the outlet passage (16a), the outlet passage extending downwardly from the air space (31) to the exterior of the container.

21.     A liquid feed device as claimed in claim 1, characterized in that the outlet passage extends downwardly from the air space and the liquid transferring means comprise means (270, 283-285, 288, 309) located above the outlet passage (280, 308) for effecting condensation of the vapour of the liquid and dripping the condensed liquid into the outlet passage (280, 308).

22.     A liquid feed device as claimed in claim 1, characterized by means (314, 315) for introducing air into the container (307) above the main body of liquid.

FIG. 1

FIG. 2

FIG. 3

FIG. 4

FIG. 5

FIG. 6

FIG. 7

FIG. 8

0078114

FIG. 9

FIG. 10

FIG. 11

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | A 61 L 9/12 |
| A | GB-A- 28 279 (W. SCHWARZHAUPT) (AD1910) * Whole document * | 1,2 | B 65 D 25/38 |
| | --- | | |
| D,A | US-A-3 157 319 (G. SCHWEINBACHER) * Whole document * | 1,3,13 -16 | |
| | --- | | |
| D,A | US-A-1 596 791 (A.L. BALL) * Whole document * | 2-4 | |
| | --- | | |
| D,A | US-A-1 349 703 (E.R. WILLIAMS) * Whole document * | 2-4,8 | |
| | ----- | | |

| TECHNICAL FIELDS SEARCHED (Int. Cl. ³) |
|---|
| A 61 L B 65 D |

The present search report has been drawn up for all claims

| Place of search THE HAGUE | Date of completion of the search 08-02-1983 | Examiner FLETCHER A.S. |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503 03.82